# EUROPÄISCHE PATENTSCHRIFT

(11) **EP 2 462 919 B1**
(45) Veröffentlichungstag und Bekanntmachung des Hinweises auf die Patenterteilung: **16.08.2017**
(21) Anmeldenummer: 11190065.0
(22) Anmeldetag: 22.11.2011
(51) Int. Cl.: A61K 8/46, A61K 8/49, A61K 8/69, A61Q 19/08, A61Q 17/04

(54) **Synergistische Wirkstoffkombination zum Schutz der Haut vor IR-Strahlung**
Synergistic active agent combination for protecting the skin from IR radiation
Combinaison de principes actifs synergétiques pour la protection de la peau contre le rayonnement IR

(30) Priorität: 13.12.2010 DE 102010062915
(43) Veröffentlichungstag der Anmeldung: 13.06.2012
(73) Patentinhaber: Henkel AG & Co. KGaA, 40589 Düsseldorf (DE)
(72) Erfinder: Welß, Thomas, 40591 Düsseldorf (DE); Holtkötter, Olaf, 50354 Hürth (DE); Emond, Eliane, 69006 Lyon (FR); Dickhof, Susanne, 41748 Viersen (DE); Janßen, Frank, 41470 Neuss (DE)

(56) Entgegenhaltungen:
- EP-A1- 1 002 533
- EP-A1- 1 591 105
- EP-A1- 1 721 601
- EP-A2- 1 803 435
- EP-A2- 2 241 305
- EP-A2- 2 243 464
- DE-A1-102004 014 613
- DE-A1-102009 037 537
- DE-A1-102009 039 393
- DE-A1-102009 045 981

## Beschreibung

Die vorliegende Anmeldung betrifft die therapeutische Verwendung einer synergistischen Wirkstoffkombination in topischen dermatologischen Zusammensetzungen zum Schutz der Haut vor Infrarot-Strahlung (IR-Strahlung).

Seit langem ist bekannt, dass der ultraviolette Anteil der Sonnenstrahlung eine Haut schädigende Wirkung hat und wesentlich zur extrinsischen Hautalterung beiträgt.

Neuere wissenschaftliche Untersuchungen haben gezeigt, dass auch IR-Strahlung zur Schädigung von Hautzellen führen kann, obwohl diese Strahlungsart weniger energiereich ist als UV-Strahlung. Laut diesen Studien führt die Bestrahlung der Haut mit IR-Strahlung im Wellenlängenbereich von 760-1400 nm zu einer Aktivierung des MAP-Kinase-Signalwegs (mitogen-activated protein-Kinase, MAPK) und zu einer vermehrten Expression der Matrixmetalloproteinase MMP-1, einer Kollagenase, in den Fibroblasten (J. Krutmann, Hautarzt 2003, 54, 809 - 817). Mit der gesteigerten Expression von MMP-1 geht ein verstärkter Abbau des Kollagens einher, das einen wesentlichen Bestandteil der extracellulären Matrix (ECM) in der Dermis darstellt und dessen Verlust eine Erschlaffung der Haut und die Bildung von Fältchen und Falten bewirkt.

Die Verbesserung des Erscheinungsbildes alternder Haut ist ein wesentliches Ziel kosmetischer Antiage-Pflegeprodukte. Im Stand der Technik sind bereits zahlreiche Wirkstoffe bekannt, die der Expression von MMP-1 entgegenwirken und bei regelmäßiger topischer Applikation das Auftreten von Hautalterungserscheinungen, wie Falten und Fältchen, verzögern können. Zum Schutz der Haut vor der schädigenden Wirkung der UV-Strahlung sind bereits gut wirksame Lichtschutzfiltersubstanzen bekannt. Aus EP 1591105 A1 ist die Verwendung diverser Antioxidantien, darunter Taurin, zum Schutz der Haut vor IR-Strahlung bekannt. Dennoch besteht ein ständiger Bedarf an weiteren Wirkstoffen und Wirkstoffkombinationen zur wirksamen Behandlung von Hautalterungserscheinungen. Besonders interessant sind dabei solche Wirkstoffe und Wirkstoffkombinationen, die möglichst früh in Prozesse, die der Hautalterung zu Grunde liegen, eingreifen und diese stoppen oder zumindest verzögern.

Der vorliegenden Erfindung lag die Aufgabe zugrunde, alternative Antiage-Zubereitungen bereitzustellen, die die Haut vor den negativen Folgen der IR-Strahlung schützen.

Es wurde nun festgestellt, dass eine Wirkstoffkombination aus Taurin und ausgewählten Dialkylchromanen oder Dialkylchromenen den Kollagenabbau in der Haut, insbesondere den durch IR-Bestrahlung induzierten Kollagenabbau, verringert.

Gegenstand der vorliegenden Anmeldung ist daher die therapeutische Verwendung einer zur topischen Applikation auf die Haut geeigneten Zusammensetzung, enthaltend a) Taurin (2-Aminoethansulfonsäure) oder ein Salz des Taurins, und b) zusätzlich zu a) mindestens einen Wirkstoff, ausgewählt aus 6,7-disubstituierten 2,2-Dialkylchromanen und 2,2-Dialkylchromenen der allgemeinen Formeln (I) und (II), 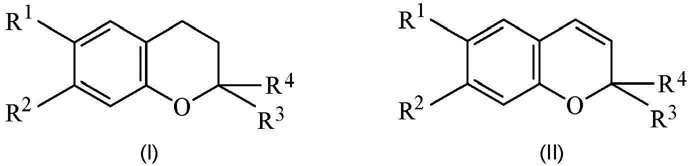 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, zur Verringerung des Kollagenabbaus in der Haut, insbesondere des durch IR-Bestrahlung induzierten Kollagenabbaus.

Bevorzugt verwendete 6,7-disubstituierte 2,2-Dialkylchromane und 2,2-Dialkylchromene der allgemeinen Formeln (I) und (II) sind dadurch gekennzeichnet, dass die Substituenten R¹ und R² unabhängig voneinander ausgewählt sind aus einer OH-Gruppe, einer Methoxy-Gruppe und einer CF₃CH₂O-Gruppe. In einer besonders bevorzugten Ausführungsform sind R¹ und R² unabhängig voneinander ausgewählt aus einer OH-Gruppe und einer Methoxy-Gruppe. In einer außerordentlich bevorzugten Ausführungsform stellen R¹ eine OH-Gruppe und R² eine Methoxy-Gruppe dar.

Die Substituenten R³ und R⁴ stellen unabhängig voneinander eine C₁-C₄-Alkylgruppe dar. Unter C₁-C₄-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind R³ und R⁴ unabhängig voneinander ausgewählt aus einer Methyl-, Ethyl-, n-Propyl-, Isopropyl- und einer n-Butyl-Gruppe. Besonders bevorzugt stellen R³ und R⁴ unabhängig voneinander eine Methyl- oder Ethylgruppe dar. Außerordentlich bevorzugt ist, dass R³ und R⁴ identisch sind. Weiterhin ist außerordentlich bevorzugt, dass R³ und R⁴ unabhängig voneinander eine Methylgruppe darstellen.

Erfindungsgemäß bevorzugt werden die 6,7-disubstituierten 2,2-Dialkylchromane verwendet. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist beispielsweise unter dem Handelsnamen Lipochroman-6 von der Firma Lipotec S.A. erhältlich.

Die 6,7-disubstituierten 2,2-Dialkylchromane oder -chromene der allgemeinen Formeln (I) oder (II) sind vorzugsweise in einer Gesamtmenge von 0,001 - 5 Gew.-%, bevorzugt 0,01 - 2 Gew.-%, besonders bevorzugt 0,05 - 1 Gew.-% und außerordentlich bevorzugt 0,1 bis 0,5 Gew.-%, jeweils bezogen auf die gesamte erfindungsgemäß verwendete Zusammensetzung, enthalten.

Taurin wird auch als 2-Aminoethansulfonsäure bezeichnet. Erfindungsgemäß geeignet ist Taurin sowohl in der undissoziierten Säureform als auch in der zwitterionischen Form (H₃N⁺-C₂H₄-SO₃⁻) und in Form seiner Salze, der Taurinate. Taurin oder Taurinate sind vorzugsweise in einer Gesamtmenge von 0,001 - 5 Gew.-%, bevorzugt 0,01 - 3 Gew.-%, besonders bevorzugt 0,1 - 2 Gew.-% und außerordentlich bevorzugt 0,5 bis 1 Gew.-%, jeweils umgerechnet auf den Gehalt an undissoziierter Säure und bezogen auf die gesamte erfindungsgemäß verwendete Zusammensetzung, enthalten.

Die erfindungsgemäß verwendeten Zusammensetzungen enthalten bevorzugt weitere Hilfsstoffe, die dem Fachmann als zur topischen Applikation auf die Haut geeignet bekannt sind. Dazu zählen beispielsweise konditionierende Wirkstoffe. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆-₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone. Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäß verwendete Zusammensetzung.

Bevorzugt liegen die erfindungsgemäß verwendeten kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonders bevorzugten Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Fettsäureester von Zuckern und Zuckeralkoholen wie Sorbit, Polyglycerine und Polyglycerinderivate, bevorzugt Polyglyceryl-2-dipolyhydroxystearat (z. B. das Handelsprodukt Dehymuls^{®} PGPH) und Polyglyceryl-3-diiso-stearat (z. B. das Handelsprodukt Lameform^{®} TGI) sowie lineare und verzweigte C₈-C₃₀-Fettsäuren und deren Na-, K-, Ammonium-, Ca-, Mg- und Zn-Salze.

Die erfindungsgemäßen oder erfindungsgemäß verwendeten Zusammensetzungen enthalten die Emulgatoren bevorzugt in Mengen von 0,1 bis 25 Gew.-%, besonders bevorzugt 0,5 - 15 Gew.-%, bezogen auf die gesamte Zusammensetzung.

In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel R¹ - O - R² kann zu lamellar-flüssigkristallinen Strukturen in der Emulsion führen, die besonders günstige Effekte auf die Wiederherstellung der lamellaren Struktur der Haut haben können. Besonders bevorzugte erfindungsgemäße oder erfindungsgemäß verwendete Zusammensetzungen sind daher dadurch gekennzeichnet, dass sie in Form einer lamellar-flüssigkristalline Strukturen aufweisenden Öl-in-Wasser-Emulsion vorliegen. Liposomen stellen keine lamellar-flüssigkristallinen Strukturen im Sinne der vorliegenden Anmeldung dar.

In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen. Genau so gut lassen sich aber auch niedrig-viskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.

Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{©} NS und Simulgel^{©} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Überraschend wurde festgestellt, dass Liposomen die der erfindungsgemäßen Verwendung zu Grunde liegenden Effekte zu blockieren scheinen. Liposomen werden vor allem aus Phospholipiden, beispielsweise Sojalecithin, Ei-Lecithin und Kephalinen, gebildet.

Ein weiterer Gegenstand der vorliegenden Anmeldung ist daher eine zur topischen Applikation auf die Haut geeignete Zusammensetzung, enthaltend a) Taurin (2-Aminoethansulfonsäure) oder ein Salz des Taurins, und b) zusätzlich zu a) mindestens einen Wirkstoff, ausgewählt aus 6,7-disubstituierten 2,2-Dialkylchromanen und 6,7-disubstituierten 2,2-Dialkylchromenen der allgemeinen Formeln (I) und (II), wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, die dadurch gekennzeichnet ist, dass keine Liposomen enthalten sind. Bevorzugt sind derartige erfindungsgemäße und erfindungsgemäß verwendete Zusammensetzungen frei von Liposomen, Phospholipiden, Lecithinen und Kephalinen.
Bezüglich weiterer bevorzugter Ausführungsformen der erfindungsgemäßen Zusammensetzungen gilt mutatis mutandis das zu den erfindungsgemäßen Verwendungen Gesagte.

### Experimenteller Teil

### 1. In vitro Assays hinsichtlich der Expression typischer Altersmarker in kultivierten Fibroblasten nach Substanzgabe und Infrarotbestrahlung

Methode: Monolayer-Fibroblasten wurden 24 Stunden vor und 24 Stunden nach Infrarot-Bestrahlung mit Lipochroman (Dimethylmethoxy Chromanol, 0,005 Gew.-%), Taurin (2-Aminoethansulfonsäure 0,005 Gew.-%) und Kombinationen beider Substanzen behandelt. Im Folgenden wurde die Genexpression der zwei Hautalterungsmarker Kollagenase MMP-1, das hinsichtlich der Hautalterung wichtigste Kollagen abbauende Enzym, und des spezifischen Inhibitors der Kollagenase MMP-1, TIMP-1 (tissue inhibitor of metalloproteinase 1), untersucht. Die Genexpressionswerte wurden im Folgenden auf die Werte der jeweiligen unbehandelten Kontrolle relativiert. Als Kontrollen wurden Fibroblasten analysiert, die ausschließlich mit Infrarot bestrahlt wurden.

Die Messergebnisse sind in der nachstehenden Tabelle zusammengestellt.

| | **Gene Expression versus Sham control** | | |
|---|---|---|---|
| | MMP-1 * | TIMP-1 * | MMP1 / TIMP-1 Ratio |
| mit Infrarot A bestrahlt | 1,2 | 0,95 | 1,2 |
| mit Infrarot A bestrahlt + 0,005 % Lipochroman-6 | 1,75 | 1,65 | 2,7 |
| mit Infrarot A bestrahlt + 0,05 % Taurin | 2,05 | 1,1 | 1,6 |
| mit Infrarot A bestrahlt + 0,005 % Lipochroman-6 + 0,05 % Taurin | 0,55 | 1 | 0,95 |

| | | | |
|---|---|---|---|
| * Regulierung mRNA (x-fach der Sham-Kontrolle) | | | |

Die Bestrahlung mit Infrarot-Strahlen aus dem Wellenlängenbereich von 760-1400 nm führte in diesem Experiment zu einer leicht gesteigerten Expression der Kollagenase MMP-1. Auch nach einer Behandlung mit Lipochroman-6 allein oder mit Taurin allein führte die Bestrahlung zu erhöhten Expressionswerten. Demgegenüber steht überraschender Weise eine signifikante Verminderung der Genaktivität nach Infrarotbestrahlung und der Behandlung mit der Kombination aus Lipochroman-6 und Taurin.

Die Bestrahlung mit Infrarot-Strahlen führt zu einer leichten Reduktion von TIMP-1. Die Bestrahlung der mit Lipochroman-6 behandelten Fibroblasten führte hingegen zu einer starken Expressionssteigerung, während Taurin und die kombinierte Behandlung zu einer leichten Steigerung der Expression führte.

Entscheidend für die Balance des Auf- und Abbaus von Kollagen in der Dermis ist das Verhältnis zwischen MMP-1 und TIMP-1. Ist dieses Verhältnis größer als der Vergleichswert, so kann von einem vermehrten Abbau durch die Kollagenase ausgegangen werden, ist das Verhältnis kleiner, so wird der Abbau vermehrt verhindert und neues Kollagen kann synthetisiert werden.
Diese Ergebnisse verdeutlichen, dass erst durch die Kombination von Taurin mit Lipochroman-6 eine positive Auswirkung auf die Kollagensynthese-Balance nach Stressung mit Infrarot-Strahlung erreicht wird.

### 2. Formulierungsbeispiele

### 2.1. Öl-in-Wasser-Emulsion

| | |
|---|---|
| Caprylic/Capric Triglyceride | 10,00 |
| Glycerin | 14,00 |
| Dicaprylyl Carbonate | 4,00 |
| Arachidyl Alcohol | 2,00 |
| Behenyl Alcohol | 0,50 |
| Butyrospermum Parkii Butter | 5,00 |
| Hydroxyethyl Urea (ex Hydrovance) | 5,34 |
| Arachidyl Glucoside | 0,25 |
| Isododecane | 2,00 |
| Panthenol | 1,00 |
| Tocopheryl Acetate | 0,50 |
| Carbomer | 0,70 |
| Phenoxyethanol | 0,70 |
| Sodium Acrylate/Sodium Acryloyldimethyl Taurate Copolymer | 0,60 |
| Parfum | 0,30 |
| Polyisobutene | 0,40 |
| Urea | 0,30 |
| Ethylparaben | 0,10 |
| Methylparaben | 0,10 |
| Sodium Hydroxide | 0,10 |
| Sorbitan Oleate | 0,03 |
| Caprylyl/Capryl Glucoside | 0,10 |
| Hexyl Salicylate | 0,02 |
| Ammonium Lactate | 0,09 |
| alpha-2-Ethylhexylglycerin | 0,50 |
| Dimethylmethoxy Chromanol | 0,01 |
| 2-Aminoethanesulfonic Acid | 0,01 |
| Aqua | ad 100,00 |

### 2.2. Öl-in-Wasser-Emulsion mit UV-Filtern

| | |
|---|---|
| Octocrylene | 5,00 |
| Homosalate | 3,00 |
| Glycerin | 7,00 |
| Butyl Methoxydibenzoylmethane | 3,00 |
| 1,6-Hexanediol | 2,00 |
| Isopropylpalmitat | 5,50 |
| Phenylbenzimidazole Sulfonic Acid | 4,00 |
| Propylheptyl Caprylate | 1,00 |
| Silica | 3,00 |
| Potassium Cetyl Phosphate | 3,00 |
| Arginine | 2,00 |
| Behenyl Alcohol | 0,50 |
| Hydrogenated Palm Glycerides | 1,50 |
| Ammonium Acryloyldimethyltaurate/VP Copolymer | 1,00 |
| Tocopheryl Acetate | 0,50 |
| Phenoxyethanol | 1,00 |
| Parfum | 0,40 |
| Acrylates/C10-30 Alkyl Acrylate Crosspolymer | 0,80 |
| Sodium Hydroxide | 0,40 |
| Ethylparaben | 0,20 |
| Methylparaben | 0,20 |
| Xanthan Gum | 0,20 |
| Tetrasodium EDTA | 0,10 |
| Bis-Ethylhexyl Hydroxydimethoxy Benzylmalonate | 0,30 |
| Tocopherol | 0,30 |
| Dimethylmethoxy Chromanol | 0,01 |
| 2-Aminoethanesulfonic Acid | 1,00 |
| Aqua | ad 100,00 |

### 2.3. Öl-in-Wasser-Emulsion, nach dem Phaseninversionstemperatur-Verfahren hergestellt (PIT-Emulsion)

| | |
|---|---|
| Emulgade SE-PF | 5,00 |
| Ceteareth-12 | 3,00 |
| Cetearyl Isononanoate | 5,00 |
| Diethylhexylcarbonat | 10,00 |
| Phenoxyethanol | 0,50 |
| Parfüm | 0,20 |
| Glycerin 86% pflanzlich | 8,00 |
| Hexandiol-1,6 | 1,00 |
| Hydrovance | 3,00 |
| Propandiol-1,2 | 1,00 |
| Dimethylmethoxy Chromanol | 0,01 |
| 2-Aminoethanesulfonic Acid | 0,10 |
| Wasser, vollentsalzt | ad 100,00 |

**Eingesetzte Rohstoffe**

| | | |
|---|---|---|
| Emulgade SE-PF | Glyceryl Stearate, Ceteareth-20, Ceteareth-12, Cetearyl Alcohol, Cetyl Palmitate | Cognis |
| Hydrovance | Aqua, N,N'-Bis-(2-hydroxyethyl)harnstoff (Hydroxyethyl urea/ 50 Gew.-%), Harnstoff, Ammoniumlactat | Akzo Nobel |

## Patentansprüche

1. Verwendung von a) Taurin (2-Aminoethansulfonsäure) oder eines Salzes des Taurins, und b) zusätzlich zu a) mindestens eines Wirkstoffes, ausgewählt aus 6,7-disubstituierten 2,2-Dialkylchromanen und 6,7-disubstituierten 2,2-Dialkylchromenen der allgemeinen Formeln (I) und (II), 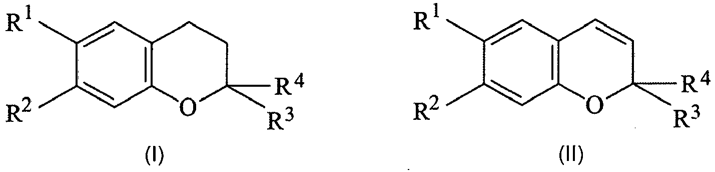 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, zur Herstellung einer zur topischen Applikation auf die Haut geeigneten Zusammensetzung, die den Kollagenabbau in der Haut verringert.

2. Verwendung gemäß Anspruch 1, **dadurch gekennzeichnet, dass** der Kollagenabbau durch InfrarotBestrahlung induziert wurde.

3. Verwendung gemäß Anspruch 1 oder 2, **dadurch gekennzeichnet, dass** R¹ und R² unabhängig voneinander ausgewählt sind aus einer OH-Gruppe und einer Methoxy-Gruppe.

4. Verwendung gemäß Anspruch 1, 2 oder 3, **dadurch gekennzeichnet, dass** R¹ eine OH-Gruppe und R² eine Methoxy-Gruppe darstellen.

5. Verwendung gemäß Anspruch 1, 2, 3 oder 4, **dadurch gekennzeichnet, dass** R³ und R⁴ unabhängig voneinander ausgewählt sind aus einer Methyl-, Ethyl-, n-Propyl-, Isopropyl- und einer n-ButylGruppe.

6. Verwendung gemäß Anspruch 1, 2, 3, 4 oder 5, **dadurch gekennzeichnet, dass** R³ und R⁴ unabhängig voneinander eine Methylgruppe darstellen.

7. Verwendung gemäß Anspruch 1, 2, 3, 4, 5 oder 6, **dadurch gekennzeichnet, dass** das 6,7-disubstituierte 2,2-Dialkylchroman der allgemeinen Formel (I) ausgewählt ist aus 2,2-Dimethyl-6-hydroxy-7-methoxy-chroman.

8. Verwendung gemäß Anspruch 1, 2, 3, 4, 5, 6 oder 7, **dadurch gekennzeichnet, dass** Taurin oder Taurinate in einer Gesamtmenge von 0,001 - 5 Gew.-%, bevorzugt 0,01 - 3 Gew.-%, besonders bevorzugt 0,1 - 2 Gew.-% und außerordentlich bevorzugt 0,5 bis 1 Gew.-%, jeweils umgerechnet auf den Gehalt an undissoziierter Säure und bezogen auf die gesamte Zusammensetzung, enthalten sind.

9. Verwendung gemäß Anspruch 1, 2, 3, 4, 5, 6, 7 oder 8, **dadurch gekennzeichnet, dass** die 6,7-disubstituierten 2,2-Dialkylchromane oder -chromene der allgemeinen Formeln (I) oder (II) in einer Gesamtmenge von 0,001 - 5 Gew.-%, bevorzugt 0,01 - 2 Gew.-%, besonders bevorzugt 0,1 - 1 Gew.-% und außerordentlich bevorzugt 0,2 bis 0,5 Gew.-%, jeweils bezogen auf die gesamte Zusammensetzung, enthalten sind.

10. Zur topischen Applikation auf die Haut geeignete Zusammensetzung, enthaltend a) Taurin (2-Aminoethansulfonsäure) oder ein Salz des Taurins, und b) zusätzlich zu a) mindestens einen Wirkstoff, ausgewählt aus 6,7-disubstituierten 2,2-Dialkylchromanen und 6,7-disubstituierten 2,2-Dialkylchromenen der allgemeinen Formeln (I) und (II), 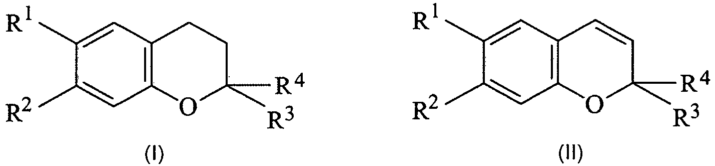 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, **dadurch gekennzeichnet, dass** keine Liposomen enthalten sind.

## Claims

1. The use of a) taurine (2-aminoethanesulfonic acid) or a salt of taurine, and b), in addition to a), at least one active ingredient selected from 6,7-disubstituted 2,2-dialkyl chromanes and 6,7-disubstituted 2,2-dialkyl chromenes of general formulas (I) and (II), 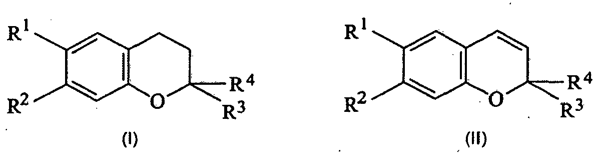 wherein R¹ and R² independently of one another represent an OH group, a methoxy group or a CF₃CH₂O group, and R³ and R⁴ independently of one another represent a C₁-C₄ alkyl group, for producing a composition that is suitable for topical application to the skin and reduces the breakdown of collagen in the skin.

2. The use according to claim 1, **characterized in that** the breakdown of collagen was induced by infrared irradiation.

3. The use according to claim 1 or 2, **characterized in that** R¹ and R² independently of one another are selected from an OH group and a methoxy group.

4. The use according to claim 1, 2, or 3, **characterized in that** R¹ represents an OH group and R² represents a methoxy group.

5. The use according to claim 1, 2, 3 or 4, **characterized in that** R³ and R⁴ independently of one another are selected from a methyl group, ethyl group, n-propyl group, isopropyl group and an n-butyl group.

6. The use according to claim 1, 2, 3, 4 or 5, **characterized in that** R³ and R⁴ independently of one another represent a methyl group.

7. The use according to claim 1, 2, 3, 4, 5 or 6, **characterized in that** the 6,7-disubstituted 2,2-dialkyl chromane of general formula (I) is selected from 2,2-dimethyl-6-hydroxy-7-methoxy-chromane.

8. The use according to 1, 2, 3, 4, 5, 6 or 7, **characterized in that** taurine or taurinates is/are contained in a total amount of 0.001-5 wt.%, preferably 0.01-3 wt.%, more preferably 0.1-2 wt.% and exceptionally preferably 0.5 to 1 wt.%, in each case converted to the content of undissociated acid and based on the total composition.

9. The use according to claim 1, 2, 3, 4, 5, 6, 7 or 8, **characterized in that** the 6,7-disubstituted 2,2-dialkyl chromanes or chromenes of general formulas (I) or (II) are contained in a total amount of 0.001-5 wt.%, preferably 0.01-2 wt.%, more preferably 0.1-1 wt.% and exceptionally preferably 0.2 to 0.5 wt.%, in each case based on the total composition.

10. Composition suitable for topical application to the skin, containing a) taurine (2-aminoethanesulfonic acid) or a salt of taurine, and b), in addition to a), at least one active ingredient selected from 6,7-disubstituted 2,2-dialkyl chromanes and 6,7-disubstituted 2,2-dialkyl chromenes of general formulas (I) and (II), 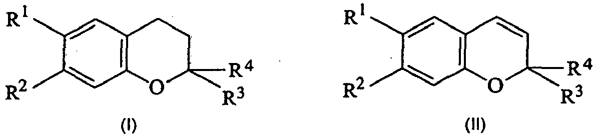 wherein R¹ and R² independently of one another represent an OH group, a methoxy group or a CF₃CH₂O group, and R³ and R⁴ independently of one another represent a C₁-C₄ alkyl group,
**characterized in that** no liposomes are contained.

## Revendications

1. Utilisation de a) taurine (acide 2-aminoéthanesulfonique) ou d'un sel de taurine, et b) en complément à a) d'au moins un principe actif, sélectionné parmi les 2,2-dialkylechromanes 6,7-disubstitués et les 2,2-dialkylechromènes 6,7-disubstitués des formules générales (I) et (II), 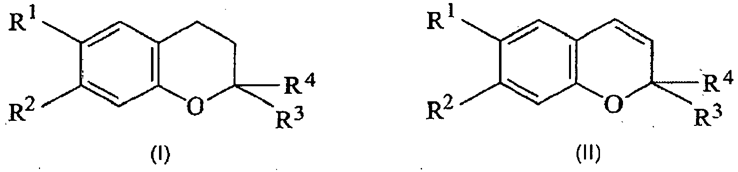 dans laquelle R¹ et R² représentent indépendamment l'un de l'autre un groupe OH, un groupe méthoxy ou un groupe CF₃CH₂O et R³ et R⁴ représentent indépendamment l'un de l'autre un groupe alkyle en C₁-C₄, pour la fabrication d'une composition adéquate pour application topique sur la peau, qui réduit la dégradation du collagène dans la peau.

2. Utilisation selon la revendication 1, **caractérisée en ce que** la dégradation du collagène a été induite par rayonnement infrarouge.

3. Utilisation selon la revendication 1 ou 2, **caractérisée en ce que** R¹ et R² sont sélectionnés indépendamment l'un de l'autre à partir d'un groupe OH et d'un groupe méthoxy.

4. Utilisation selon la revendication 1, 2 ou 3, **caractérisée en ce que** R¹ représente un groupe OH et R² représente un groupe méthoxy.

5. Utilisation selon la revendication 1, 2, 3 ou 4, **caractérisée en ce que** R³ et R⁴ sont sélectionnés indépendamment l'un de l'autre à partir d'un groupe méthyle, éthyle, n-propyle, isopropyle et n-butyle.

6. Utilisation selon la revendication 1, 2, 3, 4 ou 5, **caractérisée en ce que** R³ et R⁴ représentent indépendamment l'un de l'autre un groupe méthyle.

7. Utilisation selon la revendication 1, 2, 3, 4, 5 ou 6, **caractérisée en ce que** le 2,2-dialkylechromane 6,7-disubstitué de la formule générale (I) est sélectionné parmi le 2,2-diméthyl-6-hydroxy-7-méthoxy-chromane.

8. Utilisation selon la revendication 1, 2, 3, 4, 5, 6 ou 7, **caractérisée en ce que** la taurine ou le taurinate sont présents dans une quantité totale de 0,001 - 5 % en poids, préférablement 0,01 - 3 % en poids, très préférablement 0,1 - 2 % en poids et on ne peut plus préférablement 0,5 à 1 % en poids, respectivement converti par rapport à la teneur en acide non-dissocié et rapporté à l'ensemble de la composition.

9. Utilisation selon la revendication 1, 2, 3, 4, 5, 6, 7 ou 8, **caractérisée en ce que** le 2,2-dialkylechromane/ ou chromène 6,7-disubstitué des formules générales (I) ou (II) sont présents dans une quantité totale de 0,001 - 5 % en poids, préférablement 0,01 - 2 % en poids, très préférablement 0,1 - 1 % en poids et on ne peut plus préférablement 0,2 à 0.5 % en poids, respectivement rapporté à l'ensemble de la composition.

10. Pour l'application topique sur la peau de la composition adéquate, contenant a) de la taurine (acide 2-aminoéthanesulfonique) ou d'un sel de la taurine, et b) en complément à a) d'au moins un principe actif, sélectionné parmi les 2,2-dialkylechromanes 6,7-disubstitués et les 2,2-dialkylechromènes 6,7-disubstitués des formules générales (I) et (II), 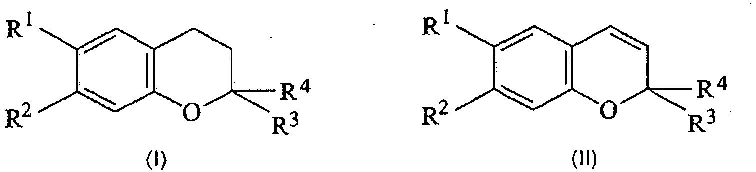 dans laquelle R¹ et R² représentent indépendamment l'un de l'autre un groupe OH, un groupe méthoxy ou un groupe CF₃CH₂O et R³ et R⁴ représentent indépendamment l'un de l'autre un groupe alkyle en C₁-C₄, **caractérisé en ce qu'**aucun liposome n'est présent.
